# EUROPEAN PATENT APPLICATION

(11) **EP 4 779 011 A1**
(43) Date of publication of application: **22.07.2026**
(21) Application number: 26152272.6
(22) Date of filing: 16.01.2026
(51) Int. Cl.: C12M 1/32, C12M 1/00

(54) **INTEGRATED VENTED LID ASSEMBLY AND METHOD**

(30) Priority: 17.01.2025 US 202563746746 P; 07.01.2026 US 202619442887
(71) Applicant: Scientific Plastic Products, Inc., Oceanside, California 92054 (US)
(72) Inventor: Ellis, Samuel A., Oceanside, 92054 (US); Landaverde, Elio, Oceanside, 92054 (US); Caustill, Joseph S., Oceanside, 92054 (US); Hingorani, Kishan G., Oceanside, 92054 (US)
(74) Representative: CMS Cameron McKenna Nabarro Olswang LLP

(57) **Abstract**

A well plate cover apparatus and method of manufacture and usage complementary to and used with well plates used in a variety of laboratories for a variety of different experiments involving small scales of samples. The well plate cover comprises a rectangular cover member comprising a generally flat upper surface and an opposed lower surface. The apparatus includes a plurality of openings formed in the lower surface of the rectangular member, each opening leading to a passage that extends through the cover member and through the interior of a protrusion that extends upwardly from the flat upper surface. A gas permeable membrane is positioned over the top opening of the protrusion for fluid communication through the passage. Additionally, the well plate cover apparatus further comprises a plurality of guiding members extending downwardly from the cover member to fit between wells of a well plate.

## Description

### CROSS-REFERENCE TO RELATED APPLICATIONS

The present application claims the benefit of U.S. Provisional Application No. 63/746,746 filed January 17, 2025, the contents of which are expressly incorporated herein by reference.

### STATEMENT RE: FEDERALLY SPONSORED RESEARCH/DEVELOPMENT

Not Applicable

### BACKGROUND

### 1. Technical Field

The present disclosure relates generally to the field of facilitating biological, particularly cellular growth within a laboratory setting. More particularly, the present disclosure relates to an improved cover for well plates that are used in a laboratory for cellular growth including a method of manufacture of the same and a method of robotic placement of the cover on a well plate.

### 2. Related Art

Biology laboratories use standard well plates and laboratory automation systems for cell cultures. In order for viable cultures to grow optimally, these well plates often need to be enclosed via a cover or lid that allows for the proper exchange of gas for the cell or microbe type being cultured while simultaneously minimizing contamination and optimizing evaporation. Some cultures require the utilization of an orbital shaker table wherein the culture is agitated for an increased or optimal yield. In many instances, the well plates include separate distinct chambers for growing multiple cultures simultaneously and it is critical to avoid material from one chamber being introduced into other chambers of the well plate. Orbital shaker tables can potentially introduce cross-contamination between culture wells by agitating the contents of the well plate such that contents from one culture well can be undesirably introduced into an adjacent or other culture well on the same well plate, making well covers a necessity. The current methods for covering well plates have deficiencies that could lead to undesirable consequences such as contamination or include mechanical complexities that result in excess cost for the consumer or user of the well plate cover.

For example, pierceable well plate seals such as those made of foil or plastic film may introduce unintended contamination from the environment, cross-contamination from other culture wells, or a rate of undesired evaporation. Well plate seals that are not pierced do not allow for gas exchange. Hard polystyrene covers and cover glass often simply rest atop the well plates without creating a proper fit, which could potentially introduce contaminants, especially cross-contaminants when used on an orbital shaker table. There are also cover and clamp systems that do include a sealable cover with membranes that allow for gas exchange while minimizing contaminants, but the system is reliant on a vertical force applied by a clamping system and may not be compatible with standard laboratory automation systems such as robotic systems. In covers where gas exchange membranes are flush with the top of the cover the location of the membrane could potentially create contamination and blockage of the membrane due to the splashing of material in a culture well onto the membrane, especially with the use of an orbital shaker table.

Thus, there exists a need in the art for a device and method of manufacturing and using a cost and space-effective well plate cover that allows for proper gas exchange while also minimizing evaporation and contamination, with or without the use of an orbital shaker table and a laboratory automation system. In addition, there exists a need in the art for a sterile single use well plate cover to minimize contamination within the wells and help ensure the use of a sterile well plate cover.

### BRIEF SUMMARY

The subject matter described herein demonstrates a device and method of manufacturing and using a well plate cover apparatus having a rectangular member in which there is an upper surface and a lower surface. There are a multitude of openings located on the lower surface which extend upwards into a passage, through a protrusion with upwardly extending side walls, leading to a corresponding top opening offset above the upper surface. A gas permeable membrane made of PTFE, PVDF, or nylon is positioned between the top opening of the protrusion and a cap that engages the side walls of the protrusion via ribbing on the interior walls of the cap and interfering ribbing on the side walls of the protrusion. The cap contains vents and spokes to hold the membrane in place and allow for fluid communication through the membrane into the passage. When not press fit on the protrusion, the membrane is configured to be held between the spokes and ribbing on the interior of the cap.

Further, the rectangular member has a multitude of integrally formed features designed to keep the well plate cover stable when fit atop a well plate. The rectangular member has a lip extending downwardly from the perimeter designed to sizably fit around the perimeter of the top of a well plate. The rectangular member has well inserts that extend downwardly from the lower surface sized to fit within the wells of a well plate. The rectangular member further has downwardly extending divider walls designed to fit between wells of a well plate. The rectangular member also has downwardly extending guiding members designed to fit between wells of a well plate in the areas where the corners of four different wells are adjacent to each other.

These features mentioned above have additional functions that allow the well plate cover to be used in a variety of functions. The well inserts may allow any agitated liquid to fall back into the well instead of out of the wells, minimizing cross-contamination of nearby wells. The membrane being elevated above the rectangular member between the cap and the top opening of the protrusion may minimize the amount of agitated liquid allowed to splash onto the membrane, minimizing membrane blockage and promoting uninterrupted fluid communication to the passage. Further, the elevated membrane and well inserts allow for a higher volume of working culture or liquid in the wells when compared to other well plates on the market. The cap having spokes and vent holes holds the membrane in place while permitting fluid communication to the passage.

The well plate cover is made compatible with standard laboratory automation systems, while also allowing for a gravity fit that may remain undisturbed when used with an orbital shaker table. The guiding members allow for the well plate cover to create the proper gravity fit, even if the cover is not precisely placed atop a well plate. The features and mechanisms of the disclosed device allow the cover to have a gravity fit to a well plate that minimizes contamination from the environment, cross-contamination between wells, and loss of working culture, especially when used with an orbital shaker table. Additionally, utilizing a gravity fit instead of a hermetic seal allows the well plate cover to be placed atop a well plate and removed from the well plate by a laboratory automation system. The use of such a well plate cover may also decrease the need for repeat experiments and thus, be more cost effective, compared to other types of well plate covers.

A method disclosed herein is the manufacture of the well plate cover apparatus. The well plate cover apparatus has a rectangular member that is injection molded and made of a hard polymer-based material such as polypropylene. The well plate cover apparatus has a multitude of integrally formed features, including but not limited to a multitude of projections that have interfering ribbing on the upper portion of the upwardly extending exterior sidewalls. The next step is forming a multitude of caps. The caps are formed of a hard polymer-based material such as polypropylene and have a multitude of features including but not limited to interior side walls, exterior side walls, ribbing on the interior sidewalls, and a top surface which has spokes and vent holes. The next step is cutting a membrane formed of PTFE, PVDF, or nylon so that the membrane fits sizable inside the cap between the ribbing on the interior walls and the underside of the spokes of the top surface. The ribbing of the cap is then press fit onto the interfering ribbing of the protrusions on the rectangular member to form the well plate cover apparatus.

A further method disclosed is the placement and removal of the well plate cover apparatus on a well plate. A robotic arm of an automation system, such as the Fluent^{®} Automation Workstation with the Robotic Gripper Arm made by Tecan Group Ltd. of Männedorf, Switzerland may engage the sides of the rectangular member. The next step is the placement of the well plate cover apparatus atop a well plate wherein downwardly extending guiding members assist in the placement of the well plate cover over a well plate. The well plate cover apparatus positioned in this manner has the ability to contain liquid within the walls of the wells of a well plate while using the high speed shaking of an orbital shaker table via a gravity fit, but it is not affixed so tightly on a well plate to limit it from being robot friendly. A further step may comprise removal of the well plate cover from a well plate via a robotic arm of a laboratory automation system which may be easily removed from the well plate due to the resting gravity placement on a well plate.

### BRIEF DESCRIPTION OF THE DRAWINGS

These and other features and advantages of the various embodiments disclosed herein will be better understood with respect to the following description and drawings, in which like numbers refer to like parts throughout, and in which:
FIG. 1 shows a top perspective view of a well plate cover apparatus affixed atop a well plate;
FIG. 2 shows a top exploded perspective view of the components of the well plate cover apparatus above a well plate;
FIG. 3 shows a bottom exploded perspective view of the components of the well plate cover apparatus above a well plate;
FIG. 4 shows a top plan view of the well plate cover apparatus;
FIG. 5 shows a side cross sectional view of the well plate cover apparatus affixed atop a well plate along line 5-5 shown in FIG. 4;
FIG. 6 shows a close-up view of a portion of the cross sectional view of FIG. 5;
FIG. 7 shows a perspective view of the sectional view of FIG. 6; and
FIG. 8 shows a side cross sectional view of the well plate cover apparatus affixed atop a well plate along line 8-8 shown in FIG. 4.

Common reference numerals are used throughout the drawings and the detailed description to indicate the same elements.

### DETAILED DESCRIPTION

The detailed description set forth below in connection with the appended drawings is intended as a description of certain embodiments of a device for an integrated vented lid assembly method of assembly and use and is not intended to represent the only forms that may be developed or utilized, nor are the described methods the only methods that could be employed. The description sets forth the various structure and/or functions in connection with the illustrated embodiments, but it is to be understood, however, that the same or equivalent structure and/or functions may be accomplished by different embodiments that are also intended to be encompassed within the scope of the present disclosure. It is further understood that the use of relational terms such as first and second, and the like are used solely to distinguish one entity from another without necessarily requiring or implying any actual such relationship or order between such entities.

In some embodiments, the numbers expressing dimensions, quantities, quantiles of ingredients, properties of materials, and so forth, used to describe and claim certain embodiments of the disclosure are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the disclosure are approximations, the numerical values set forth in the specific examples are reported as precisely as practicable. The numerical values presented in some embodiments of the disclosure may contain certain errors necessarily resulting from the standard deviation found in their respective testing measurements.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

As used herein, and unless the context dictates otherwise, the term "coupled to" is intended to include both direct coupling (in which two elements that are coupled to each other contact each other) and indirect coupling (in which at least one additional element is located between the two elements). Therefore, the terms "coupled to" and "coupled with" are used synonymously.

Unless the context dictates the contrary, all ranges set forth herein should be interpreted as being inclusive of their endpoints, and open-ended ranges should be interpreted to include commercially practical values. Similarly, all lists of values should be considered as inclusive of intermediate values unless the context indicates the contrary.

The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the disclosure and does not pose a limitation on the scope of the claimed inventive subject matter. No language in the specification should be construed as indicating any non-claimed element essential to the practice of the inventive subject matter.

Groupings of alternative elements or embodiments of the inventive subject matter disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

It should be apparent to those skilled in the art that many more modifications besides those already described are possible without departing from the inventive concepts herein. The inventive subject matter, therefore, is not to be restricted except in the scope of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

The following discussion provides many example embodiments of the inventive subject matter. Although each embodiment represents a single combination of inventive elements, the inventive subject matter is considered to include all possible combinations of the disclosed elements. Thus if one embodiment comprises elements A, B, and C, and a second embodiment comprises elements B and D, then the inventive subject matter is also considered to include other remaining combinations of A, B, C, or D, even if not explicitly disclosed. Various objects, features, aspects and advantages of the inventive subject matter will become more apparent from the following detailed description of preferred embodiments, along with the accompanying drawing figures in which like numerals represent like components.

Referring now to the drawings, wherein the showings are for purposes of illustrating preferred embodiments of the present disclosure, and are not for purposes of limiting the same, there is depicted a well plate cover apparatus 10 for covering a well plate 12 for use in the culturing of cells in a microbiological laboratory setting. The well plate cover apparatus 10 may also be interchangeably referred to as the well plate cover, the cover, the well plate lid, or the lid. When reference is made to culturing cells, a cell culture, a desired cell culture, a culture, or the like, there is equal applicability to all types of cell cultures, including but not limited to bacterial cell cultures, mammalian cell cultures, and insect cell cultures, as well as all other unicellular organism or multicellular organism cell cultures. The covering of well plates for use in culturing cells is a useful and oftentimes necessary step for creating viable and high-yield cultures. Further, using a well plate cover suited to the needs of a specific cell culture is associated with several advantages, including facilitating increased cell growth and increased viability. For instance, including a filter in a well plate cover can allow for optimal gas exchange in the wells while reducing contaminants and further, including an elevated filter may prevent blockage of the filter, especially when used with an orbital shaker table.

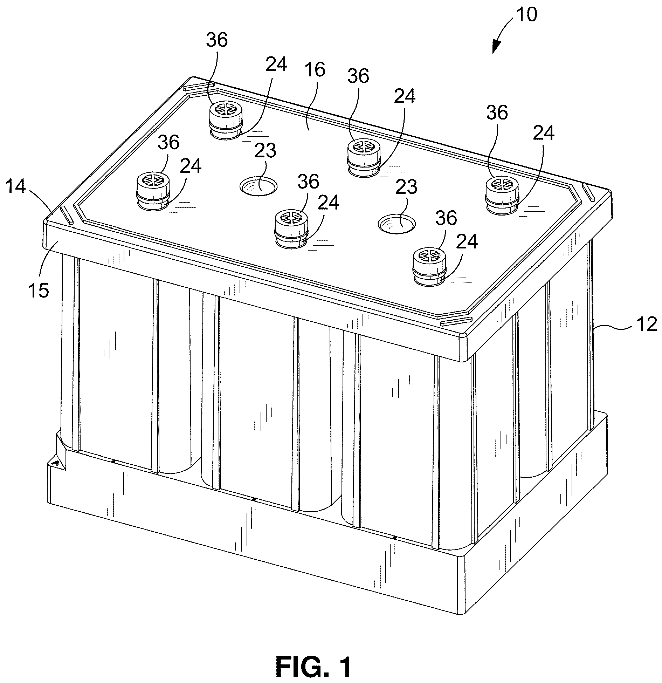

Referring to Figures 1 to 3, Figure 1 is a top perspective view of a well plate cover apparatus 10, or well plate cover affixed atop a well plate 12. The well plate 12 may be a standard well plate as widely used in laboratories. These well plates 12 are known and have been in use for many years. For example, the well plate 12 shown in the figures is referred to in the industry as a six well plate.

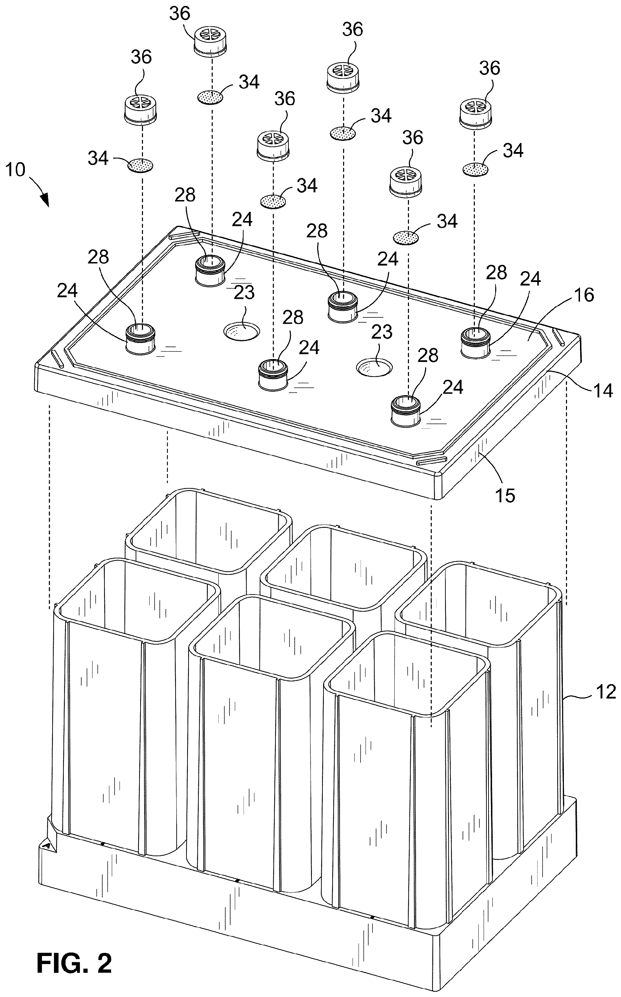

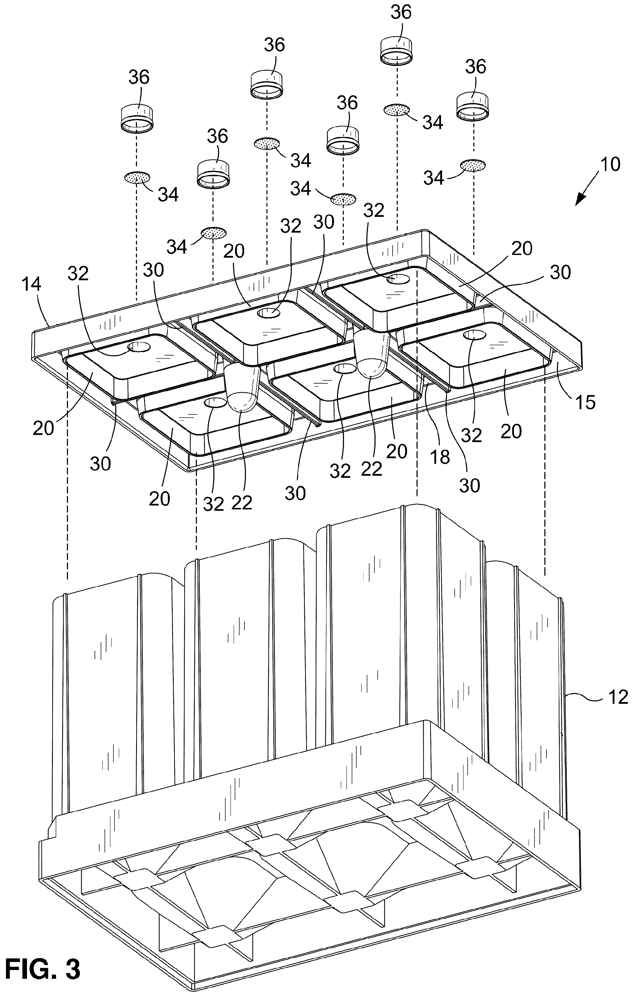

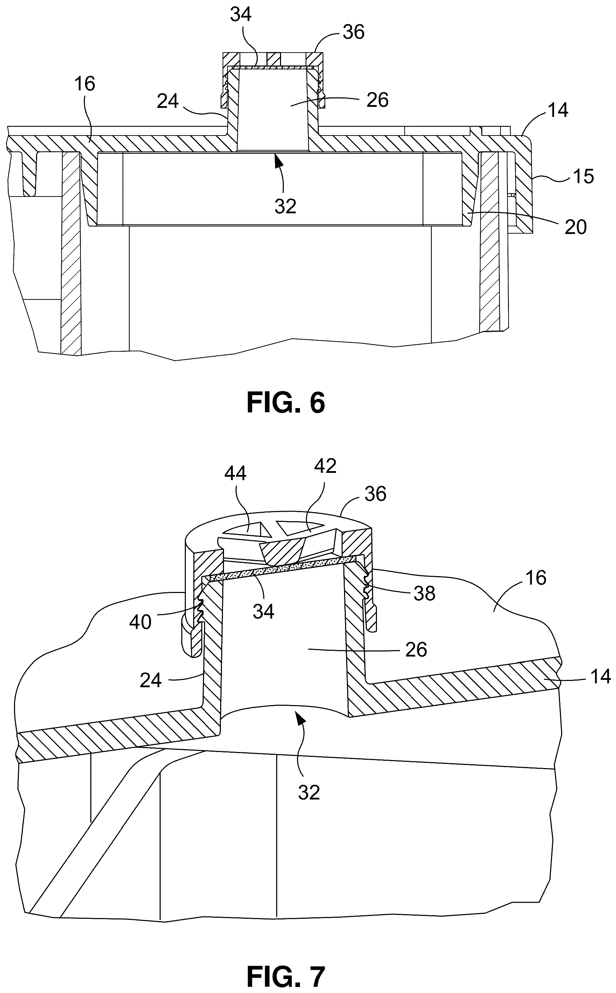

Figure 2 is a top exploded perspective view of the components of the well plate cover apparatus 10 shown in Figure 1, above a well plate 12. Figure 3 is the exploded well plate cover of Figure 2 from a bottom perspective view. These components depicted in Figure 2 include, but are not limited to, a rectangular member 14 having a generally planar upper surface 16 and a lip 15 extending downwardly around the perimeter of the member 14, a plurality of upwardly extending chimneys 24 with openings 28 and the complementarily fitting membranes 34 and caps 36 located above the chimneys 24. Figure 3 further depicts the rectangular member 14 having a bottom surface 18, wherein tapered well inserts 20 extend downwardly to engage the wells of a well plate, and guiding members 22 and divider walls 30 extend downwardly to engage the gaps between wells of a well plate 12. Openings 32 in the bottom surface 18 open into the chimneys 24 to define a passage 26 (shown in Figures 6-7) through the member 14 extending to the openings 28 shown in Fig. 2.

The well plate cover apparatus 10, as shown in Figures 1 to 3 may comprise an injection molded rectangular member 14 (as best shown in Figure 2) formed of a unitary piece of rigid polymer-based material having features including, but not limited to, a generally planar upper surface 16, a plurality of upward extending chimneys 24 extending from the upper surface 16, a bottom surface 18, wherein well tapered well inserts 20 extend downwardly from the bottom surface 18 along with guiding members 22 (shown in Figure 3) extending downwardly from the member 14 to engage gaps in the well plate 12. The member 14 also includes a lip 15 extending around the perimeter of the member 14. The upper surface 16 may also be referred to as the top surface or the outer surface. The lower surface 18 may also be referred to as the bottom surface or the inner surface. The well plate cover 10 shown in Figures 1 through 8 depict the cover 10 for a six well plate, but it has been contemplated that the well plate cover 10 may further be adapted to fit any standard well plate configuration with any number of wells, i.e., a two well plate, a twenty-four well plate, a ninety-six well plate, or any other well plate known in the art. The well plate cover 10 dimensions would be sized and configured to engage well plates that are different than the six well plate 12 depicted in the disclosure, but would include like features as depicted in the well plate cover 10 described herein.

The cover 10 has an integrally formed lip 15 that extends downwardly from the perimeter of the member 14 to cover a portion of a well plate 12. The lip 15 is configured to be sizably fit around a well plate 12 to prevent the cover 10 from accidentally becoming dislodged from a well plate 12, while still allowing for automated laboratory systems to easily apply and remove the well plate cover 10. It is contemplated by this disclosure that the lip 15 may further have a tapered inner edge, a ridge extending from the inner edge of the lip to a well plate 12, a gasket made of rubber or another material known in the art affixed to the inner wall of the lip 15, or another analogous feature to adapt the well plate cover 10 to have varying degrees of strength of affixation securable to a well plate 12 to accommodate varying types of automation or manual placement or removal of the cover 10 from the well plate 12. In the disclosure herein, the lip 15 offers little or no resistance to the removal and placement of the cover 10 to the well plate 12 in order to accommodate automated methods such as robotic systems that may place and remove the cover 10 on the well plate 12. The member 14 may be formed of a polymer-based material such as polypropylene or polyethylene although it is contemplated by the present disclosure that other rigid or semirigid material such as silicon or rubber may be deployed to form the member 14. It is generally contemplated by this disclosure that the cover 10 may be a single use sterile disposable item thus providing additional protection against the use of a non-sterile well plate cover. It is also contemplated by this disclosure that instead of using polypropylene or other plastic material, a material such as glass, ceramic or metal, which is able to be sterilized using an autoclave, may be used.

The bottom surface 18 may have integrally formed well inserts 20, that extend downwardly from the bottom surface 18 to engage a portion of the well of a well plate 12, as shown in Figure 3. The well inserts 20 have downwardly extending sidewalls that are designed to have the largest cross section at the point that it is attached to the bottom surface and taper to the smallest at its bottommost point to create a gravity fit inside of a well of a well plate 12. The well inserts 20 may extend far enough into the well of a well plate 12 that any residual liquid that may be splashed are contained within the well. Material may be splashed onto the well inserts 20 or the bottom surface 18 due to agitation caused by, for example, an orbital shaker table and are designed to allow any material to be able drip back into the well instead of splashing out of the well. The gravity fit of the well inserts 20 may create a secure enough fit as to not allow liquid out of the well, but the fit may not be so secure that the well plate cover 10 cannot be easily applied to and removed from a well plate 12 by a laboratory automation system. It has been contemplated that the inserts 20 may further have an attached O-ring formed of rubber, silicone, neoprene, nitrile or other materials known in the art to act as a gasket and create a hermetic seal inside of the well.

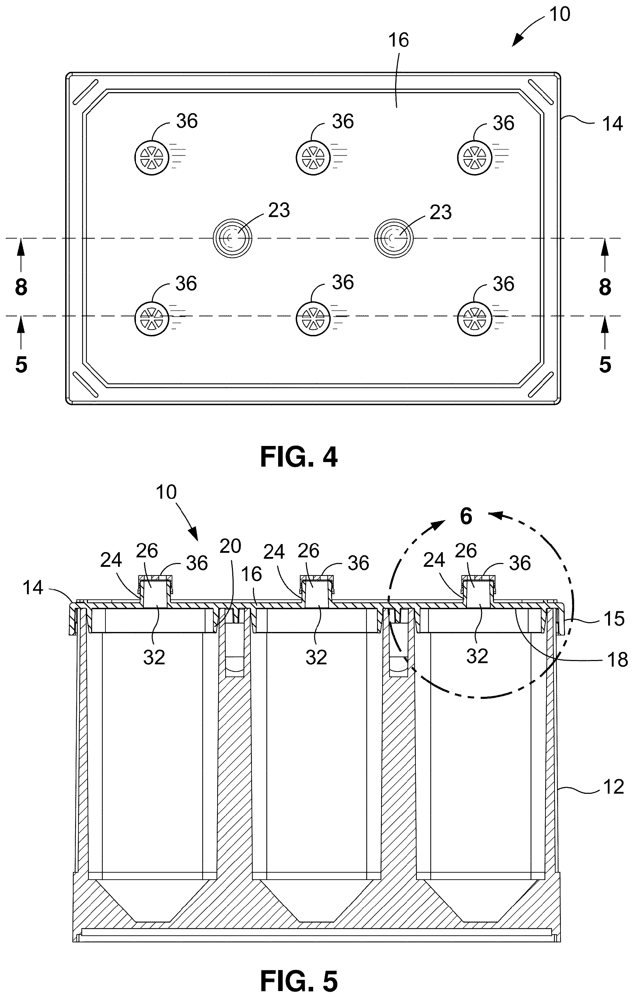

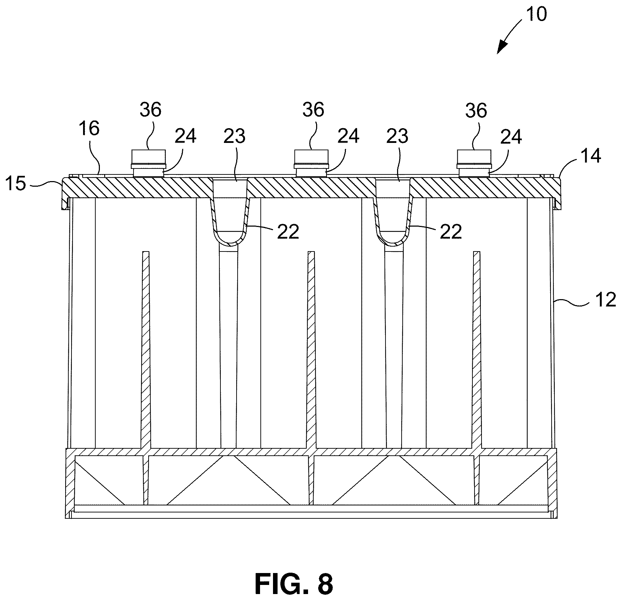

Referring to Figures 4 and 8, Figure 4 depicts a top plan view of the well plate cover apparatus 10 with dashed lines 5-5 and 8-8 indicating where cross-sectional views are to be depicted. Figure 8 is a cross-sectional view along line 8-8 of Figure 4.

The member 14, additionally, may have hollow, generally conical shaped downwardly extending guiding members, or guiding members 22, as shown in Figure 3. The guiding members 22 are integrally formed into the member 14, forming voids 23 in the member 14 that are visible from above the well plate cover 10 and are shown in Figures 1, 2, and 4. A cross-sectional view depicting the guiding members 22 and voids 23 as they fit inside a well plate 12 can be seen in Figure 8. The guiding members 22 and corresponding voids 23 extend downwardly from the member 14 to fit between four adjacent wells in a well plate. The guiding members 22 may act as a guiding apparatus to assist laboratory automation systems, such as robots, in properly placing the well plate cover 10 atop a well plate 12.

Likewise, the bottom surface 18 may have thin downwardly extending divider walls 30 designed to fit between two adjacent wells of a well plate 12. The divider walls 30 may also act as a guiding apparatus to assist laboratory automation systems in properly placing the well plate cover 10 atop to a well plate 12. The divider walls 30 are integrally formed as part of the unitary structure of member 14 into the bottom surface 18 of the cover member and may similarly be made of polypropylene or another rigid polymer-based material, or may be attached to the bottom surface 18 and made of rubber, silicone, neoprene, nitrile, or other similar materials known in the art.

Referring to Figures 4 through 7, Figure 4 depicts a top plan view of the well plate cover apparatus 10 with dashed lines 5-5 and 8-8 indicating where cross-sectional views are to be depicted. Figure 5 depicts a cross-sectional view at line 5-5. Figures 6 and 7 show a close-up, cross-sectional view of the area inside of the dashed circle in Figure 5.

The well plate cover apparatus 10 includes the rectangular member 14 having a bottom surface 18 with several bottom chimney openings 32 that extends upwardly through the member 14 to a corresponding top chimney opening 28 elevated above the top surface 16 creating an integrally formed chimney 24, shown first in Figure 1 and shown in more detail in Figures 5 to 7. The chimney 24 may also be referred to as the projection or the protrusion. The integrally formed interior of the chimney 24 may create a passage 26 situated between and including the bottom chimney openings 32 and the corresponding top chimney openings 28, first shown in Figure 2. The passage 26 may also be referred to as the channel. The passage 26, bottom chimney openings 32, and corresponding top chimney openings 28 may all share a diameter of 12mm. It is contemplated by this disclosure that the diameter of the passage 26 may be variable for different applications relating to differing types of cell cultures. The chimney 24 and integrally formed passage 26 allows for access from above the cover member through the passage 26 into a well of the well plate 12. The chimney 24 as part of the unitary member 14 integrally formed passage 26, is formed of polypropylene or another rigid polymer-based material such as polystyrene. It has been contemplated that instead of using polypropylene, a material such as metal, glass, or ceramic, which is able to be sterilized using an autoclave, may be used.

Turning to figures 6 and 7, the well plate cover apparatus 10 may have caps which may be formed of polypropylene 36 that fit over a top portion of the chimneys 24 thereby at least partially covering the top chimney openings 28. Interference ribbing, or interfering ribs 38 may be formed on outer surface of the chimney 24 adjacent the top opening of the chimney 28 with the interfering ribs 38 configured to mate with ribbing, or ribs 40 formed on the inside the cap 36 to be secured to the top of the chimney 24. The interfering ribs 38 and ribs 40 may be forcibly interlocked by press fitting the bottom side of the cap 36 onto the chimney 24 over the interference ribs 38.

The cap 36 additionally may have spokes 42 and vent holes 44 located in the top cover portion of the cap 36. A membrane 34 may be situated to sizably fit between the underside of the spokes 42 and the topmost portion of the interfering ribs 40 inside the cap 36. The membrane 34 may be situated this way so that when the cap 36 is press fit onto the chimney 24, the membrane 34 sits securely atop the passage 26 at the top opening 28 of the chimney, and underneath the spokes 42 and vent holes 44 of the cap 36. This is done so that the membrane 34 is elevated above the top surface 16 of the member 14. The membrane 34 may be made to allow gas exchange from the environment into the wells of a well plate 12 and allow gas to escape from the wells. Likewise, the spokes 42 and vent holes 44 of the cap may be sized to allow for uninhibited gas exchange through the membrane 34, while keeping it securely in place. It has been contemplated that the size and shape of the spokes 42 and vent holes 44 may be altered based upon different applications relating to different types of cell cultures.

The membranes 34 may be made of polytetrafluoroethylene (PTFE) with a sterile porosity of 0.2 µm and a diameter of 12mm. It has been contemplated that the membrane 34 may be made of a different chemically inert material such as polyvinylidene fluoride (PVDF), nylon, or another material known in the art. It has also been contemplated that the diameter and sterile porosity of the membrane 34 may be variable to suit the various needs of various types of cell cultures. The membrane 34 being located atop the chimney 24 and passage 26, elevated above the top surface 16 of the member 14 may allow for agitated liquid caused by an orbital shaker table to minimally interfere with, contaminate, or create blockage in the membrane 34. The upwardly extending passage 26 may make it more difficult for liquid to reach the membrane 34 while still allowing for gas exchange between the culture and the atmospheric air. Additionally, the combination of the membrane 34 being elevated above the top surface 16 and the well inserts 20 allow for a higher volume of liquid or working culture to be used in the wells of a well plate 12. Using the same well plate 12, standard well plate covers on the market only allow for about 20 to 30mL of liquid or working culture to be used in conjunction with an orbital shaker table, whereas the disclosed apparatus 10 allows for up to 50mL to be used in conjunction with an orbital shaker table. The ability to use more liquid or working culture may lead to a more desirable yield and increase productivity in experiments using the proposed well plate cover apparatus 10. In order for certain types of cell cultures to be viable, the cells must take in oxygen from the environment and dispel carbon dioxide into the environment; this process being referred to as gas exchange. Different types of cell cultures have different necessary rates of gas exchange to maintain cell viability.

A method disclosed herein is the manufacture of the well plate cover apparatus. The well plate cover apparatus has a rectangular member that is injection molded and made of a hard polymer-based material such as polypropylene. The well plate cover apparatus has a multitude of integrally formed features, including but not limited to a multitude of projections that have interfering ribbing on the upper portion of the upwardly extending exterior sidewalls. The next step is forming a multitude of caps. The caps are formed of a hard polymer-based material such as polypropylene and have a multitude of features including but not limited to interior side walls, exterior side walls, ribbing on the interior sidewalls, and a top surface which has spokes and vent holes. The next step is cutting a membrane formed of PTFE, PVDF, or nylon so that the membrane fits sizable inside the cap between the ribbing on the interior walls and the underside of the spokes of the top surface. The ribbing of the cap is then press fit onto the interfering ribbing of the protrusions on the rectangular member to form the well plate cover apparatus.

A further method disclosed is the placement and removal of the well plate cover apparatus on a well plate. A robotic arm of an automation system, such as the Fluent^{®} Automation Workstation with the Robotic Gripper Arm made by Tecan Group Ltd. of Männedorf, Switzerland may engage the sides of the rectangular member. The next step is the placement of the well plate cover apparatus atop a well plate wherein downwardly extending guiding members assist in the placement of the well plate cover over a well plate. The well plate cover apparatus positioned in this manner has the ability to contain liquid within the walls of the wells of a well plate while using the high speed shaking of an orbital shaker table via a gravity fit, but it is not affixed so tightly on a well plate to limit it from being robot friendly. A further step may comprise removal of the well plate cover from a well plate via a robotic arm of a laboratory automation system which may be easily removed from the well plate due to the resting gravity placement on a well plate.

The above description is given by way of example, and not limitation. Given the above disclosure, one skilled in the art could devise variations that are within the scope and spirit of the device and methods disclosed herein, including various ways of assembling, forming, or using the well plate cover described herein. Further, the various features of the embodiments disclosed herein can be used alone, or in varying combinations with each other and are not intended to be limited to the specific combination described herein. Thus, the scope of the claims is not to be limited by the illustrated embodiments.

## Claims

1. A well plate cover apparatus comprising:
a rectangular member comprising:
an upper surface and an opposed lower surface;
at least one opening formed in the lower surface; and
at least one protrusion extending upwardly from the upper surface having an interior passage and top opening positioned in spatial relation to the upper surface wherein the interior passage is in fluid communication with the at least one opening formed in the lower surface; and
a membrane positioned over the top opening of the protrusion.

2. The well plate cover apparatus of claim 1:
(a) wherein the membrane is gas permeable;
(b) further comprising a lip extending downwardly from at least a portion of the rectangular member designed to sizably fit over at least a portion of a well plate; or
(c) further comprising a well insert integrally formed on the lower surface of the rectangular member and sized to fit within an opening of a well plate.

3. The well plate cover apparatus of claim 1, wherein the at least one protrusion has upwardly extending sidewalls.

4. The well plate cover apparatus of claim 3, further comprising at least one cap sized to engage the sidewalls of the at least protrusion to at least partially cover the top opening.

5. The well plate cover apparatus of claim 4, wherein the at least one cap includes at least one vent to permit fluid communication to the passage.

6. The well plate cover apparatus of claim 5, wherein the at least one cap includes radial spokes defining a plurality of vents to permit gas exchange to the passage.

7. The well plate cover apparatus of claim 3, wherein the cap has interior and exterior side walls and wherein the interior side walls includes ribbing.

8. The well plate cover apparatus of claim 3, wherein the outer surface of the sidewalls of the protrusion includes interference ribbing.

9. The well plate cover apparatus of claim 1, wherein the membrane consists primarily of a material selected from the group consisting of PTFE, PVDF and nylon, optionally wherein the membrane is sized and configured to be held between the radial spokes and the ribbing of the at least one cap.

10. The well plate cover apparatus of claim 1, further comprising:
(a) at least one guiding member extending downwardly from at least a portion of the cover member designed to fit between wells of a well plate; or
(b) at least one divider wall extending downwardly from at least a portion of the cover member designed to fit between wells of a well plate.

11. A method for manufacture of a well plate cover apparatus comprising:
forming a rectangular member comprising:
an upper surface and an opposed lower surface;
at least one opening in the lower surface;
at least one protrusion extending upwardly from the upper surface forming an interior passage and top opening positioned in space relation to the upper surface wherein the interior passage is in fluid communication with at least one opening formed in the lower surface;
forming at least one cap;
forming at least one membrane positioned within the at least one cap; and
press fitting the at least one cap onto the at least one protrusion.

12. The method of claim 11, wherein the at least one protrusion has upwardly extending side walls.

13. The method of claim 11, wherein the at least one cap has interior and exterior side walls and wherein the interior side walls includes ribbing, optionally wherein the outer surface of the side walls of the at least one protrusion includes interference ribbing.

14. The method of claim 11, wherein the rectangular member is injection molded.

15. A well plate cover apparatus comprising:
a rectangular member comprising:
an upper surface and an opposed lower surface;
at least one opening formed in the upper surface and extending through the rectangular member; and
at least one protrusion comprising sidewalls extending from the upper surface and defining an interior passage in fluid communication with the at least one opening; and
a gas permeable membrane affixed to the at least one protrusion such that the membrane is positioned to permit the venting of gas passing through the interior passage.
